Europäisches Patentamt

European Patent Office

Office européen des brevets

(11) Publication number: **0 357 465**
**A1**

## (12) EUROPEAN PATENT APPLICATION

(21) Application number: **89309093.6**

(22) Date of filing: **30.08.89**

(51) Int. Cl.⁵: **A 61 K 31/445**
**// (A61K31/445,31:44)**

(30) Priority: **31.08.88 GB 8820578**

(43) Date of publication of application:
**07.03.90 Bulletin 90/10**

(84) Designated Contracting States:
**AT BE CH DE FR GB IT LI NL SE**

(71) Applicant: **GLAXO GROUP LIMITED**
**Clarges House 6-12 Clarges Street**
**London W1Y 8DH (GB)**

(72) Inventor: **Humphrey, Patrick Paul Anthony**
**Arcadia House Church Lane**
**Wrestlingworth Bedfordshire SG19 2EU (GB)**

**Lumley, Philip**
**7 St. John's**
**Puckeridge Hertfordshire SG11 1SY (GB)**

(74) Representative: **Skailes, Humphrey John ét al**
**Frank B. Dehn & Co. Imperial House 15-19 Kingsway**
**London WC2B 6UZ (GB)**

(54) Pharmaceutical compositions for the treatment of occlusive vascular diseases.

㊄ The use is described of both (A) [1R-[1α(Z̲),2β,3β,5α]]-(+)-7-[5-[[1,1'-biphenyl)-4-yl]methoxy]-3-hydroxy-2-(1-piperidinyl)cyclopentyl]-4-heptenoic acid or a physiologically acceptable salt, solvate or cyclodextrin complex thereof and (B) (E)-5-[[[(3-pyridinyl)[3-(trifluoromethyl)phenyl]methylen]amino]oxy]pentanoic acid either separately or in combination in the therapy or prophylaxis of occlusive vascular diseases.

Pharmaceutical compositions containing both (A) and (B) are also described.

EP 0 357 465 A1

Description

## PHARMACEUTICAL COMPOSITIONS FOR THE TREATMENT OF OCCLUSIVE DISEASES

This invention relates to improvements in the treatment of occlusive vascular diseases, and in particular to improvements in anti-thrombotic therapy.

In EP-A-256805 we reported the discovery that the thromboxane receptor blocker [1R-[1α(Z),2β,3β,5α]]-(+)-7-[5-[[(1,1'-biphenyl)-4-yl]methoxy]-3-hydroxy-2-(1-piperidinyl)cyclopentyl]-4-heptenoic acid (hereinafter referred to as Compound A) or a salt thereof will act synergistically with a thromboxane synthase inhibitor to inhibit blood platelet aggregation. A number of specific thromboxane synthase inhibitors were mentioned in EP-A-256805 and we stated therein that using the test described by Lumley & Humphrey (J. Pharmacol. Methods, 1981, 6, 153-166) with collagen as the pro-aggregatory agent we had shown that Compound A or a salt thereof (e.g. the hydrochloride salt) in the presence of thromboxane synthase inhibitors such as dazoxiben or CV4151 in isolated human whole blood acted synergistically in inhibiting collagen-induced aggregation.

EP-A-221601 describes a group of oxime-alkane carboxylic acid derivatives having thromboxane synthase inhibitory activity. One compound described and claimed therein is the compound (E)-5-[[[-5-[[[(3-pyridinyl)[3-(trifluoromethyl)phenyl]methylen]amino]oxy]pentanoic acid (hereinafter referred to as Compound B).

We have now found that in the Lumley & Humphrey test referred to above Compound A or a salt (e.g. the hydrochloride salt), solvate or cyclodextrin complex thereof acts synergistically with Compound B or a salt thereof to inhibit collagen-induced aggregation. We have also found that this combination has a better biological profile of action then the combination of Compound A or a salt thereof with dazoxiben or CV 4151. Compound A or a physiologically acceptable salt, solvate or cyclodextrin complex thereof and Compound B or a salt thereof in combination therapy are therefore of interest for use in human and veterinary medicine, more particularly for use in the treatment or prophylaxis of occlusive vascular diseases. Particular examples of occlusive vascular diseases are known in the art but include myocardial infarction, cardiac fatalities, angina (e.g. unstable angina), transient ischaemic attacks and cerebral infarction, atherosclerosis and vessel wall disease, peripheral vascular disease, nephropathy, diabetic retinopathy, postoperative thrombosis and pulmonary embolism, renal dialysis, peptic ulcer disease, peri- and postoperative complications following organ transplantation (particularly cardiac and renal), coronary artery bypass, peripheral artery bypass, angioplasty, thrombolysis and endarterectomy. Compound A or a physiologically acceptable salt, solvate or cyclodextrin complex thereof and Compound B or a salt thereof in combination therapy are also of use in the treatment or prophylaxis of cyclosporin A-induced nephrotoxicity and in the treatment of asthma and adult respiratory distress syndrome.

Thus, according to one aspect of the invention, we provide a method of treating occlusive vascular diseases in human or animal subjects which comprises administering to the patient effective amounts of Compound A or a physiologically acceptable salt, solvate or cyclodextrin complex thereof and Compound B or a physiologically acceptable salt thereof.

It will be appreciated that Compound A or a physiologically acceptable salt, solvate or cyclodextrin complex thereof and Compound B or a physiologically acceptable salt thereof may be administered to the patient either simultaneously, sequentially or in combination. When the compounds are administered separately Compound A or a physiologically acceptable salt, solvate or cyclodextrin complex thereof is preferably administered first. However, the administration should be managed such that the individual compounds will both be present at the same time in their active forms in the human or animal body.

In another aspect of the invention, therefore, we provide Compound A or a physiologically acceptable salt, solvate or cyclodextrin complex thereof and Compound B or a physiologically acceptable salt thereof in the presence of each other in the patient for use in human or veterinary medicine, more particularly for use in the treatment or prophylaxis of occlusive vascular diseases.

In a further aspect of the invention we provide a combination of Compound A or a physiologically acceptable salt, solvate or cyclodextrin complex thereof and Compound B or a physiologically acceptable salt thereof.

In a yet further aspect of the invention we provide a combination of Compound A or a physiologically acceptable salt, solvate or cyclodextrin complex thereof and Compound B or a physiologically acceptable salt thereof for use in human or veterinary medicine, more particularly for use in the treatment or prophylaxis of occlusive vascular diseases.

In another aspect of the invention we provide the use of Compound A or a physiologically acceptable salt, solvate or cyclodextrin complex thereof and Compound B or a physiologically acceptable salt thereof in the preparation of a medicament for the treatment or prophylaxis of occlusive vascular diseases in the human or animal body.

In using Compound A or a physiologically acceptable salt, solvate or cyclodextrin complex thereof and Compound B or a physiologically acceptable salt thereof according to the present invention, it is preferable to employ them in the form of pharmaceutical formulations which may be in separate formulations or in a single combined formulation. However, in the latter formulation both active ingredients must of course be stable and mutually compatable in the particular formulation employed.

The present invention therefore also provides a pharmaceutical composition which comprises Compound A or a physiologically acceptable salt, solvate or cyclodextrin complex thereof and Compound B or a physiologically acceptable salt thereof together, where desirable, with one or more pharmaceutical carriers or excipients.

In another aspect of the invention we provide a pharmaceutical composition comprising Compound A or a physiologically acceptable salt, solvate or cyclodextrin complex thereof and Compound B or a physiologically acceptable salt thereof together, where desirable, with one or more pharmaceutical carriers or excipient for use in human or veterinary medicine, more particularly for use in the treatment of prophylaxis of occlusive vascular diseases.

When Compound A or a physiologically acceptable salt, solvate or cyclodextrin complex thereof and Compound B or a physiologically acceptable salt thereof are to be used as a single combined composition, the composition may be administered in any suitable forms, particularly for oral or parenteral administration.

The compositions may take the form of, for example, tablets, capsules, powders, solutions or syrups for oral administration. The compositions may thus contain as excipients, for example, binding agents, compression aids, fillers, lubricants, disintegrants and wetting agents. Tablets may be coated in a conventional manner, for example with a suitable film-forming material such as methyl cellulose or hydroxypropylmethyl cellulose. Alternatively the tablets may be sugar coated. Liquid preparations may also contain, for example, edible oils such as peanut oil.

For parenteral administration the compositions of the invention may take a form suitable for continuous infusion. Such forms include suspensions, solutions or emulsions in oil or aqueous vehicles, which may optionally contain formulatory agents such as suspending, stabilising and/or dispersing agents. Alternatively, the active ingredients may be in powder form for reconstitution before use with a suitable vehicle, e.g. sterile pyrogen-free water. Formulations for injections may be presented in unit dosage form in ampoules, or in multi-dose containers, with an added preservative, or in a suitable container for infusion.

The compositions of the invention may be prepared according to methods well known in the pharmaceutical industry. Thus, for example, tablets may be prepared by direct compression of the active ingredients blended with appropriate excipients. Alternatively, the blend of active ingredients and excipients may first be granulated using conventional techniques and the resulting granules compressed into tablets. Tablets may be film coated with suitable film forming materials using standard techniques.

Capsules may be prepared by blending the active ingredients and excipients and then filling the blend into gelatin capsules using a suitable filling machine.

Solutions for parenteral administration may be prepared by dissolving the active ingredients in a suitable vehicle e.g. water, and adjusting the tonicity and pH of the solution as required. The solution may if desired be clarified and then filled into appropriate sized ampoules. Sterilisation may be carried either before or after filling. If desired the solution may be packed under an inert atmosphere of nitrogen.

Thus, in another aspect of the invention we provide a process for the preparation of a pharmaceutical composition which comprises mixing Compound A or a physiologically acceptable salt, solvate or cyclodextrin complex thereof and Compound B or a physiologically acceptable salt thereof together, where desirable, with one or more carriers or excipients.

As stated hereinbefore, Compound A or a physiologically acceptable salt, solvate or cyclodextrin complex thereof and Compound B or a physiologically acceptable salt thereof may be administered as two separate compositions. Compound A or a physiologically acceptable salt, solvate or cyclodextrin complex thereof will preferably be administered first and may be used in various pharmaceutical formulations for oral or parenteral administration or for administration by inhalation.

Compound A and physiologically acceptable salts and solvates thereof, methods for their preparation and formulations containing them are described in GB-B-2097397 and GB-B-2127406. Suitable salts of Compound A include acid addition salts derived from inorganic and organic acids such as hydrochlorides, hyrobromides, sulphates, phosphates, maleates, tartrates, citrates, benzoates, 2-chlorobenzoates, p-toluenesulphonates, methanesulphonates, salicylates, fumarates, lactates, hydroxynaphthalenecarboxylates (e.g. 1-hydroxy- or 3-hydroxy-2-naphthalenecarboxylates) or furoates, or salts with suitable bases such as alkali metal (e.g. sodium and potassium), alkaline earth metal (e.g. calcium or magnesium), ammonium and substituted ammonium (e.g. dimethylammonium, triethylammonium, 2-hydroxyethyl dimethylammonium, piperazine, N,N-dimethylpiperazine, piperidine, ethylenediamine and choline) salts. A preferred salt of Compound A is the hydrochloride salt which is described in GB-B-2127406.

Cyclodextrin complexes of Compound A and formulations thereof are described in our co-pending UK Patent Specification 2211737A. Formulations may be prepared in conventional manner by mixing a cyclodextrin complex of Compound A with one or more pharmaceutical carriers or excipients, for example, according to the general methods described in GB-B-2097397 and GB-B-2127406. When a liquid composition for, in particular, parenteral (e.g. intravenous) administration is required this may be prepared according to the general methods described in GB-B-2097397 and GB-B-2127406. Alternatively, liquid compositions may be prepared by mixing Compound A or the hydrochloride salt thereof with α-, or β- or γ-cyclodextrin (or a hydrate thereof) or a mixture of two or three of them together, where desirable, with one or more pharmaceutical carriers of excipients in a suitable medium such as water according to conventional methods. The molar ratio of Compound A or its hydrochloride salt with cyclodextrin in liquid composition is conveniently within the range 1:1 to 1:4, and preferably the liquid composition is an aqueous solution which contains the hydrochloride salt of Compound A and β-cyclodextrin (or a hydrate thereof) in a molar ratio of about 1:1.4.

When Compound A is used according to the present invention in the form of a cyclodextrin complex, the complex conveniently contains a molar ratio of Compound A with cyclodextrin within the range 1:1 to 1:3. The cyclodextrin within the complex may be any of α-, β- or γ-cyclodextrin or a mixture of any of two, or three of them, although preferably the complex contains β-cyclodextrin. A particularly preferred cyclodextrin complex of Compound A is the β-cyclodextrin complex in which the molar ratio of Compound A with β-cyclodextrin is about 1:1.

Cyclodextrin complexes may be prepared by dissolving Compound A or the hydrochloride salt thereof in water or an organic solvent which is miscible with water (e.g. an alcohol such as methanol) and adding to the solution a solution of α-, β- or γ-cyclodextrin (or a hydrate thereof) or a mixture of two or three of them together in water and/or an organic solvent which is miscible with water. The reaction may take place at any temperature in the range from 0° to 80°C. However, the mixture is preferably kept at room temperature and the desired complex obtained by concentrating the mixture under reduced pressure or by allowing the mixture to cool. The mixing ratio of organic solvent with water may be suitably varied according to the solubilities of the starting materials and products. Preferably 1 to 4 moles of cyclodextrin are used for each mole of Compound A or its hydrochloride salt.

It may be convenient to present Compound A or a physiologically acceptable salt, solvate or cyclodextrin complex thereof and Compound B or a physiologically acceptable salt thereof as a two container pack, each container containing one of the active ingredients. The compounds may then be admixed immediately before administration, or, if, desired, may be administered sequentially.

The invention also provides Compound A or a physiologically acceptable salt, solvate or cyclodextrin complex thereof and Compound B or a physiologically acceptable salt thereof and compositions containing them in association with instructions for their use together in the therapy or prophylaxis of occlusive vascular diseases in human or animal subjects.

Compound B and its salts and formulations thereof may be prepared by the methods described in EP-A-221601.

The relative proportions of Compound A or a physiologically acceptable salt, solvate or cyclodextrin complex thereof and Compound B or a physiologically acceptable salt thereof for use according to the present invention and the precise dose of each active ingredient to be administered, conveniently in a single composition or two separate compositions, will, of course, depend on a number of factors including, for example, the age and weight of the patient, the specific condition requiring treatment and its severity and the route of administration. However, generally a ratio by weight of about 25:1 to 1:10, in particular about 10:1 to 1:5, and especially about 1:1 of Compound A or a physiologically acceptable salt, solvate or cyclodextrin complex thereof to Compound B or a physiologically acceptable salt thereof may be used.

As stated above, the precise dose of each active ingredient to be administered will depend on a number of factors and will ultimately be at the discretion of the attendant physician or vetinarian. However, the amount of Compound A or a physiologically acceptable salt, solvate or cyclodextrin complex thereof employed according to the present invention, conveniently in the form of a composition, will preferably be in the range of 3.5 to 350mg, particularly 3.5 to 100mg per unit dose. The amount of Compound B or a physiologically acceptable salt thereof employed according to the present invention, conveniently in the form of a composition, will preferably be in the range of 5 to 500mg per unit dose. In general, the composition(s) will be administered in 1-4 doses per day.

The following Preparations and Examples are provided in illustration of the present invention and should not be construed in any way as constituting a limitation thereof. In the Examples the term 'Thromboxane A₂ Antagonist' means Compound A or a physiologically acceptable salt, solvate or cyclodextrin complex thereof, especially the hydrochloride salt. The term 'Thromboxane Synthase Inhibitor' means Compound B or a physiologically acceptable salt thereof. All temperatures are in °C.

## Preparation 1

### (a)
[1R-[1α(Z),2β,3β,5α]]-(+)-7-[5-[[(1,1'-Biphenyl)-4-yl]methoxy]-3-hydroxy-2-(1-piperidinyl)cyclopentyl]-4-heptenoate: β-cyclodextrin (1:1) complex

β-Cyclodextrin hydrate (0.954g) was nearly completely dissolved in water (35mℓ). The suspension was filtered and the filtrate added to a solution of Compound A (0.2g, Example 10 in GB-B-2097397) in methanol (10mℓ). The reaction solution was stirred at 21° for 26h to give a clear solution which was evaporated to a volume of 12mℓ when slight crystallisation began to occur. The suspension was cooled to 5° for 1h to produce a thick precipitate which was filtered off and dried to give the title compound (0.273g), m.p. >310°, darkens above 230°.

(b) The filtrate from the above experiment began to precipitate more crystalline material on standing and was therefore evaporated to leave a white solid which was dissolved in hot water (3mℓ), cooled (5°) and allowed to crystallise to give a white crystalline solid (121mg) shown by ¹H N.M.R. (DMSO) analysis to contain [1R-[1α(Z),2β,3β,5α]]-(+)-7- [5-[[(1,1'-biphenyl)-4-yl]methoxy]-3-hydroxy-2-(1-piperidinyl) cyclopentyl]-4-heptenoate : β-cyclodextrin (1:1.5) complex.

## Preparation 2

[1R-[1α(Z),2β,3β,5α]]-(+)-7-[5[[(1,1'-Biphenyl)-4-yl]methoxy]-3-hydroxy-2-(1-piperidinyl)cyclopentyl]-4-heptenoate: γ-cyclodextrin (1:1.5) complex

A solution of γ-cyclodextrin (1.09g) in water (25mℓ) was added to a solution of Compound A (0.20g) in methanol (10mℓ). The reaction solution was stirred at 21° for 26h to produce a thick white suspension. The precipitate was filtered off and dried in vacuo to leave the title compound as a white solid (0.612g), m.p. >310°, darkens above 250°.

## Example 1

### Preparation of Parenteral Injections/Infusions of the Hydrochloride Salt of Compound A

| (i) | | | |
|---|---|---|---|
| Hydrochloride salt of Compound A equivalent to 50 mg base | 54mg | 54mg | 54mg |
| β-cyclodextrin hydrate | 143mg | 166mg | 238mg |
| Sodium hydroxide solution | to pH7 | to pH7 | to pH7 |
| Water suitable for injection | to 50ml | to 50ml | to 50ml |

The hydrochloride salt of Compound A was dissolved in 35ml water suitable for injection and the β-cyclodextrin was added. This solution was titrated to pH7 with 0.02M sodium hydroxide solution and then adjusted to volume with water suitable for injection.

The solution may then be sterilised by filtration and filled into vials or ampoules.

| (ii) Hydrochloride salt of Compound A equivalent to 50mg base | 54mg |
|---|---|
| β-cyclodextrin hydrate | 166mg |
| Sodium chloride | 450mg |
| pH7.0 phosphate buffer | 2.5ml |
| Sodium hydroxide solution | to pH7 |
| Water suitable for injection | to 50ml |

The hydrochloride salt of Compound A was dissolved in approximately 25ml water suitable for injection. The β-cyclodextrin was dissolved therein and the resulting solution was titrated to pH6 with 0.02M sodium hydroxide solution and the phospate buffer added. The sodium chloride was added to the solution and the pH adjusted to pH7 with sodium hydroxide. The solution was made up to volume with water suitable for injection. A sample of this solution was filled into a glass vial which was sealed with a rubber plug and metal overseal. This was then autoclaved.

## Example 2

### Preparation of a Tablet containing both active ingredients

| | mg/tablet |
|---|---|
| Thromboxane A₂ Antagonist | 20.0 |
| Thromboxane Synthase Inhibitor | 20.0 |
| Microcrystalline cellulose | 159.0 |
| Magnesium stearate BP | 1.0 |
| Compression weight | 200.0mg |

The thromboxane A₂ antagonist and thromboxane synthase inhibitor are sieved and blended with the microcrystalline cellulose and magnesium stearate. The resulting mix is compressed on a suitable tablet machine using 8mm punches. Tablets of other strengths may be prepared by altering the ratio of the active ingredients to microcrystalline cellulose or the compression weight and using punches to suit.

The tablets may be film coated with suitable film forming materials, such as hydroxypropyl methylcellulose, using standard techniques. Alternatively the tablets may be sugar coated.

Tablets may also be prepared by other conventional methods such as wet granulation.

## Example 3

### Preparation of a Capsule containing both active ingredients

| | mg/capsule |
|---|---|
| Thromboxane A₂ Antagonist | 20.0 |
| Thromboxane Synthase Inhibitor | 20.0 |
| Starch 1500* | 159.0 |
| Magnesium stearate BP | 1.0 |
| Fill Weight | 200.0mg |

* a form of directly compressible starch

The thromboxane A₂ antagonist and thromboxane synthase inhibitor are sieved and blended with the Starch 1500 and the excipients. The mix is filled into size No. 2 hard gelatin capsules using suitable machinery. Capsules of other strengths may be prepared by altering the ratio of the active ingredients to Starch 1500 or the fill weight and if necessary changing the capsule size to suit.

## Claims

1. The use of (A) [1R-[1α(Z),2β,3β,5α]]-(+)-7-[5-[[1,1′-biphenyl)-4-yl]methoxy]-3-hydroxy-2-(1-piperidinyl)cyclopentyl]-4-heptenoic acid or a physiologically acceptable salt, solvate or cyclodextrin complex thereof and (B) (E)-5-[[[(3-pyridinyl)[3-(trifluoromethyl)phenyl]methylen]amino]oxy]pentanoic acid in the manufacture of medicaments for the use (either separately or in combination) of (A) and (B) in the presence of each other in the therapy or prophylaxis of occlusive vascular diseases in human or animal subjects.

2. The use according to claim 1 in which the compounds (A) and (B) are presented as separate compositions for said use.

3. A pharmaceutical composition which comprises both a compound (A) and a compound (B) as defined in claim 1.

4. The use or a composition according to any preceding claim, in which compound (A) is in the form of its hydrochloride salt.

5. The use or a composition according to any preceding claim in which the ratio of compound (A) to compound (B) is about 1:1 by weight.

6. The use or a composition according to any preceding claim, in which compounds (A) and (B) are in a form suitable for oral or parenteral administration.

7. The use or a composition according to claim 6, in which compounds (A) and (8) are in the form of tablets or capsules.

8. The use according to claim 2 in which the hydrochloride salt of compound (A) is in a form suitable for intravenous administration.

9. A two-container pack for use in the therapy or prophylaxis of occlusive vascular diseases, one of the containers containing a compound (A) and the other containing a compound (B) as defined in claim 1.

10. A composition according to any of claims 3 to 8, or a pack according to claim 9 or (A) or (B) as defined in claim 1 in association with instructions for the use of both (A) and (B) in the therapy or prophylaxis of occlusive vascular diseases in human or animal subjects.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 256 805 (GLAXO GROUP LTD) <br> * Page 8, lines 5-21; claims 1-5 * <br> --- | 1-10 | A 61 K 31/445// <br> (A 61 K 31/445 <br> A 61 K 31:44 ) |
| D,A | EP-A-0 221 601 (JANSSEN PHARMACEUTICA N.V.) <br> ----- | 1-10 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 15-11-1989 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)